# EUROPEAN PATENT SPECIFICATION

(11) **EP 3 678 719 B1**
(45) Date of publication and mention of the grant of the patent: **05.04.2023**
(21) Application number: 17835880.0
(22) Date of filing: 06.09.2017
(51) Int. Cl.: A61M 11/00

(54) **AEROSOL THERAPY DEVICE**
VORRICHTUNG FÜR AEROSOLTHERAPIE
DISPOSITIF DE THÉRAPIE PAR AÉROSOL

(43) Date of publication of application: 15.07.2020
(73) Proprietor: 3A Health Care S.R.L., 25017 Lonato del Garda BS (IT)
(72) Inventor: ABATE, Simone, 25015 Desenzano del Garda Brescia (IT); FRACCAROLI, Davide, 25015 Desenzano del Garda Brescia (IT)
(74) Representative: Chimini, Francesco
(86) International application number: PCT/IT2017/000186
(87) International publication number: WO 2019/049178

(56) References cited:
- WO-A1-2011/161706
- WO-A1-2015/015394
- US-A1- 2002 002 975

## Description

The present invention, as defined by the appended claims, relates to an aerosol therapy device of the type comprising an aerosol body housing a motor-compressor group and wherein suction and air delivery means are respectively provided to and from said motor-compressor group, and an ampoule for the treatment of the respiratory tract, fluidically connected to the air delivery means. The motor-compressor group comprises an electric motor and a compressor member actuatable by the electric motor to suck in and deliver air respectively through suction and air delivery means. The ampoule comprises a cup-shaped ampoule body defining a chamber for housing a medication and wherein is provided a nozzle having a delivery hole for the compressed air coming from the compressor member. A nozzle-cover, also called a 'pisper' or 'impactor', is fitted on the nozzle so as to form with the nozzle at least one channel for sucking the medication towards the delivery hole by means of the Venturi effect. The nozzle-cover is provided with a nebulizer element positioned in front of and coaxially to the delivery hole to nebulize the air-medication mixture exiting from the delivery hole.

Generally, the electric motor comprises a stator and a rotor. The stator comprises an electric winding formed by a certain number of turns which, when an alternating current runs through it, generates in a laminated core an alternating magnetic field crossed by the rotor. The alternating magnetic field causes the rotation of the rotor, which actuates the compressor member, for example eccentrically connected to the rotor of the drive shaft.

In the aerosol devices of this type currently on the market, the number of turns in the electric winding does not exceed 1800 turns, the diameter of the copper wire of the turns is equal to or greater than 0.35 mm, and the laminated core has a thickness equal to or greater than 20 mm. With a 230 V mains supply voltage, such an electric motor uses more than 0.5 A of current, for a consumption of approximately 120 VA.

Such power is required to rotate a drive shaft with a diameter of 5 mm or greater, so as to generate a pressure of more than 0.6 bar in a delivery hole of the ampoule of approximately 0.5 mm in diameter.

Usually, the internal volume of the ampule does not exceed 50 cm³.

The ampoule contains a medication, which for the purpose of the present invention also includes simple saline or other liquid which, when nebulized, helps to dissolve the mucus, and nebulizes the medication to be inhaled by the patient through a mask or an accessory for nasal irrigation.

Usually, the nebulized substance exiting the nozzle - nozzle-cover assembly is channeled to an ampoule delivery mouth, also called a mouthpiece, through a tubular duct; in the description below it is also referred to as a "chimney", which extends above and coaxially to the nozzle. For example, such tubular duct is made in a lid that closes the ampoule superiorly. WO2015/015394 describes an aerosol therapy delivery device with adjustable positive airway pressure for treatment and therapy of bronchopulmonary disease.

In some embodiments of the present invention, it is possible to make the action of the nebulized substance even more effective by selecting the size of the particles which form the nebulized substance, as a function of the area of the respiratory tract to be treated. Thus, for example, to treat the upper respiratory tract, such as the larynx and pharynx, larger particles are used than those needed to reach the lower respiratory tract, such as the bronchi and the lungs.

To obtain this particle size selection, chimneys of different heights are used to vary the distance with respect to the nozzle. In particular, longer chimneys, which completely surround the point of formation of the nebulized substance, allow mainly smaller particles to be conveyed to the dispensing mouth, as the larger, heavier particles impact on the inner lateral surface of the chimney; shorter chimneys, which are unable to surround the point of formation of the nebulized substance, have a smaller impact surface for the larger particles, which may then reach the dispensing mouth. Smaller particles, however, dissipate mostly in the chamber of the ampoule.

An object of the present invention is to propose an aerosol device of the type described above, but having more compact dimensions and less power usage.

Another object of the present invention is to provide an ampoule of the type described above that allows the effective selection of the particle size of the nebulized substance delivered.

Another object of the invention is to propose an ampoule which allows the use of chimneys of different heights without modifying the other components of the device.

A further object of the invention is to provide an ampoule which allows, with respect to the ampoules according to the prior art, the amount of residual medication, i.e. that which is not nebulized, to be reduced.

Such objects are achieved by means of an aerosol therapy device according to claim 1. The dependent claims describe preferred or advantageous embodiments of the device.

The features and advantages of the device according to the invention will, however, become evident from the description hereinafter of their preferred embodiments, provided by way of indicative and non-limiting examples, with reference to the accompanying figures, wherein:

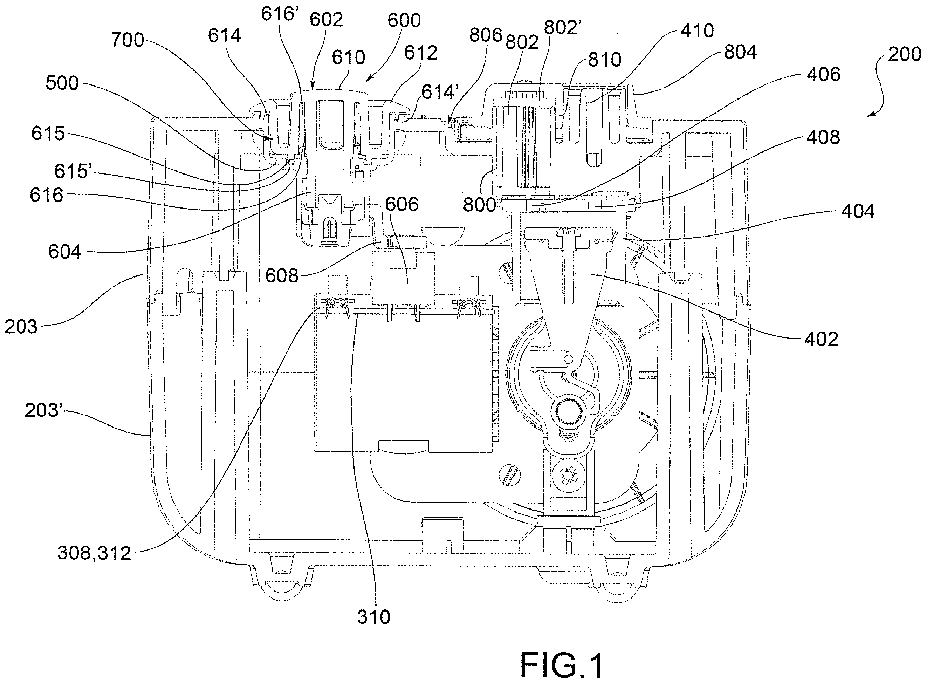

- figure 1 is an axial section of the aerosol body of the aerosol therapy device according to the invention;

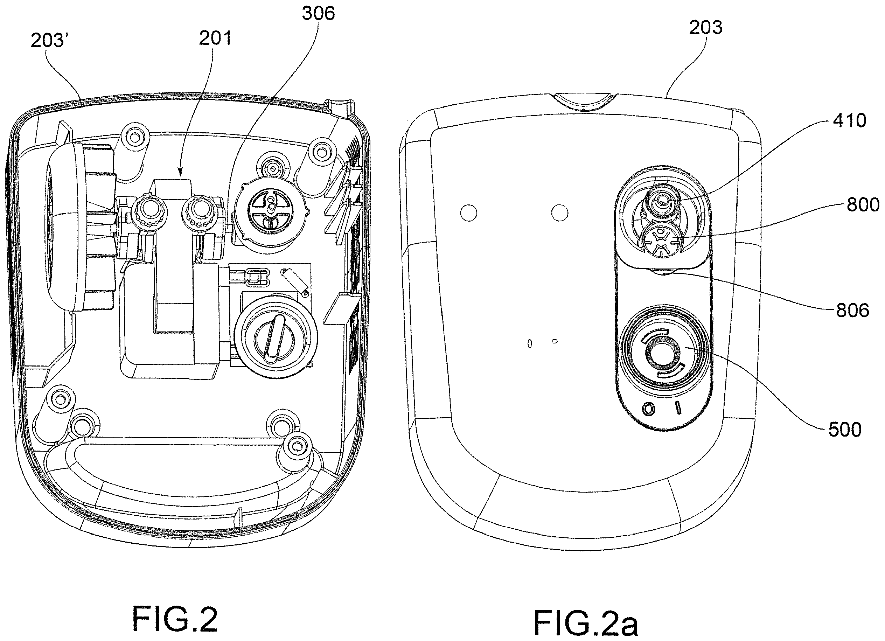

- figure 2 is a top plan view of two separate parts of the aerosol body;
- figure 3 is a perspective view of the part of the aerosol body housed in the motor-compressor group;
- figure 4 is a perspective view of the motor-compressor group;

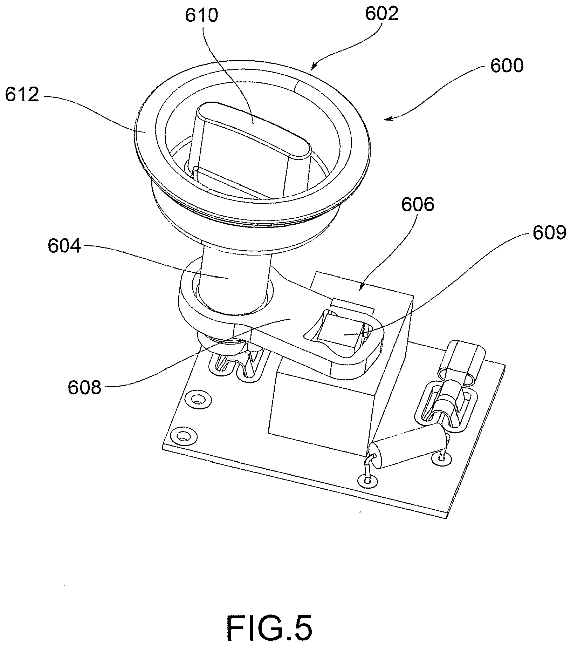

- figure 5 shows, in a perspective view, the on/off knob of the electric motor integrated with the power and control circuit board of the electric motor;

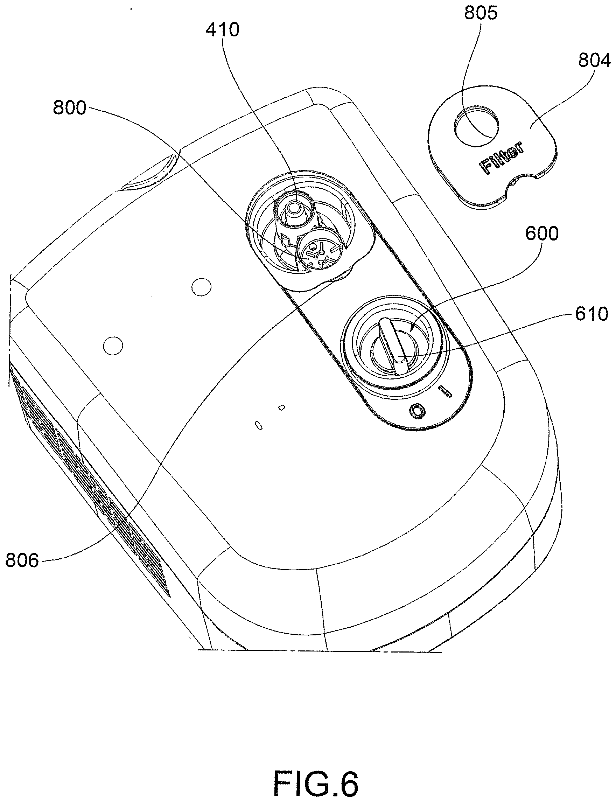

- figure 6 is an upper view of the top portion of the aerosol body, with the filter cover removed from the filter;
- figure 7 is an elevation view of an ampoule of the aerosol therapy device according to the invention in a first configuration of use;
- figure 8 is an elevation view of the ampule, in a second configuration of use;

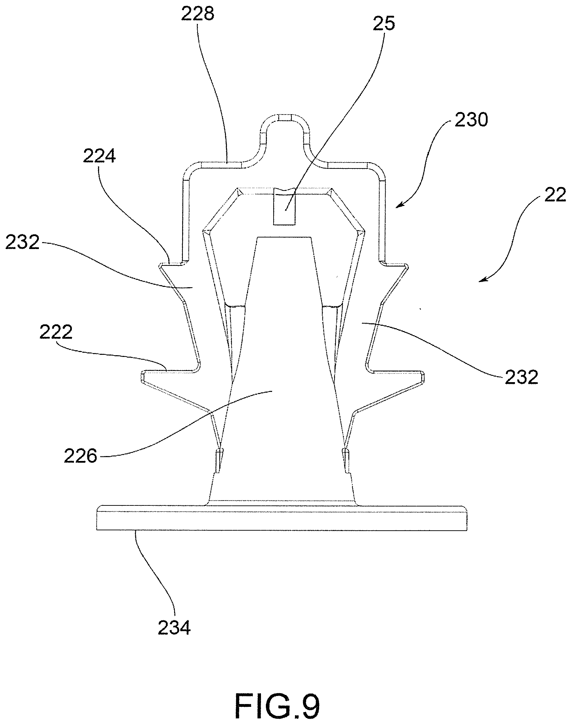

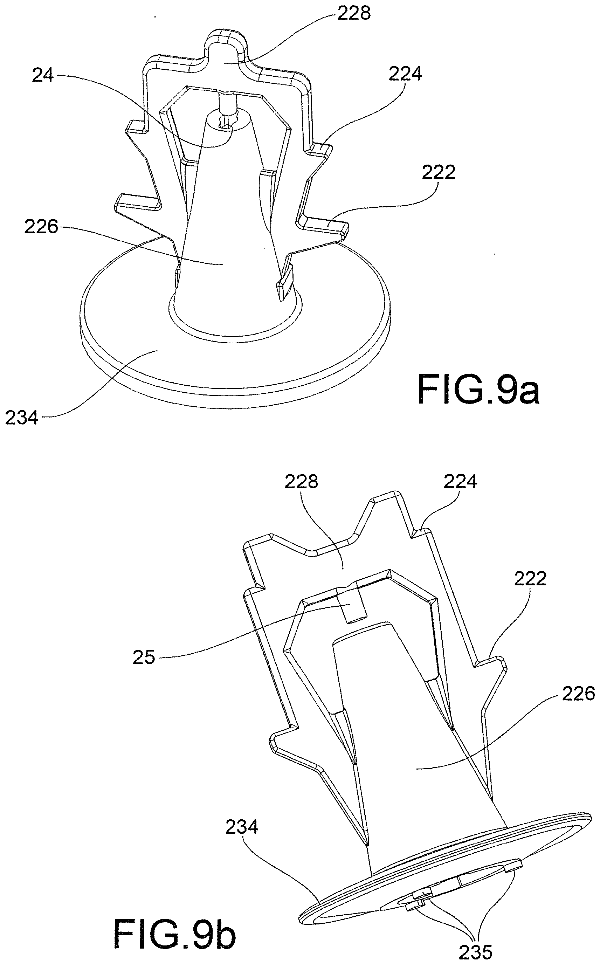

- figures 9 and 9a show an elevation view and a perspective view of the ampoule nozzle-cover according to the invention;
- figure 9b shows a perspective view from below of the nozzle-cover in one variant of embodiment;
- figures 10 and 10a schematically represent the chamber of the ampoule with the nozzle-cover, in a straight and inclined position, respectively; and

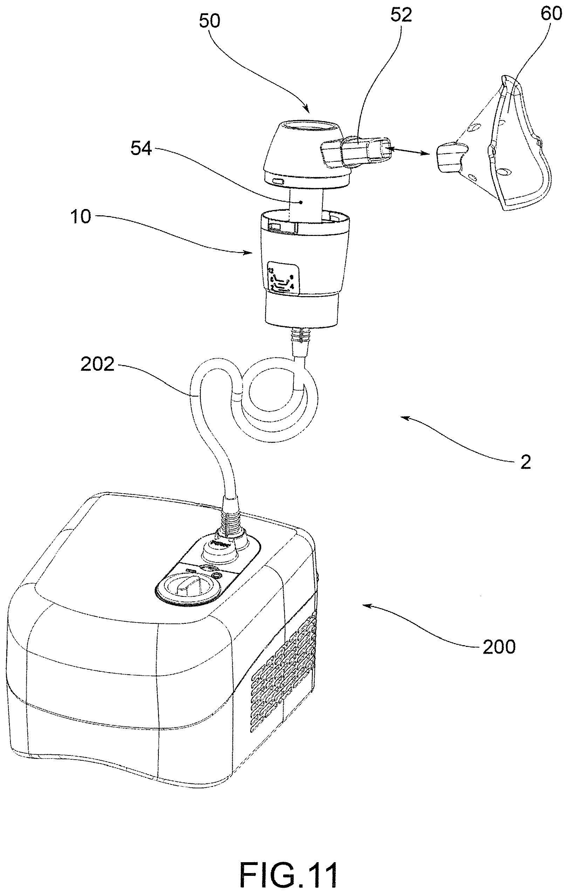

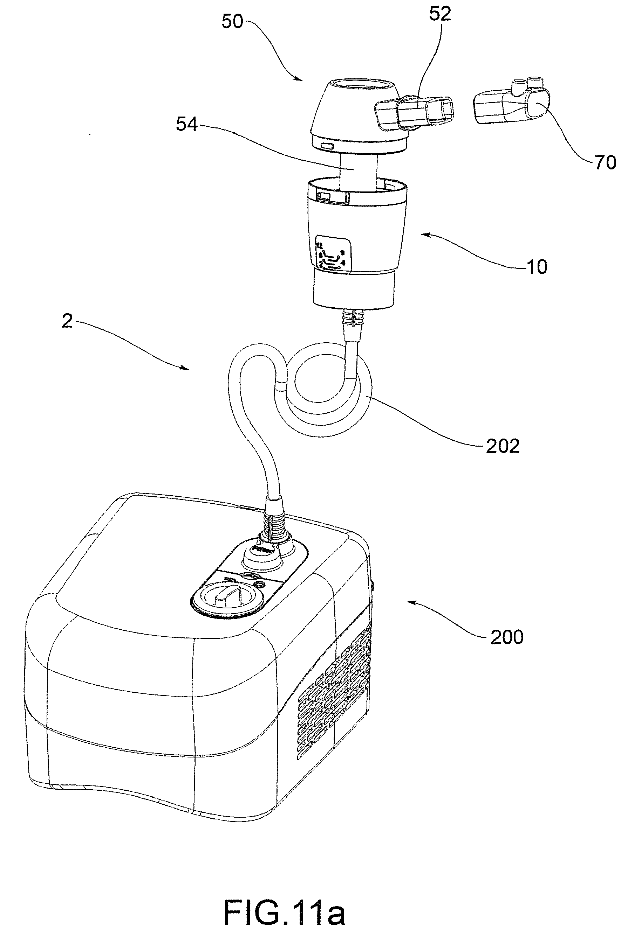

- figures 11 and 11a are two perspective views of the aerosol therapy device with the ampoule with separate components, in one configuration with an aerosol therapy mask and in a configuration with an accessory for nasal irrigation, respectively.

The elements or parts of elements in common between the embodiments described hereinafter will be indicated at the same numerical references.

In said drawings, an aerosol therapy device according to the invention has been indicated collectively at 2.

The device 2 comprises an aerosol body 200 wherein is housed a motor-compressor group 201 and wherein are provided suction and air delivery means respectively to and from said motor-compressor group and an ampoule 1 for the treatment of the respiratory tract fluidically connected to the air delivery means.

The motor-compressor group 201 comprises an electric motor 300 and a compressor member 400 actuatable by the electric motor 300 to suck in and deliver air respectively through suction and air delivery means.

The electric motor 300 is a rotary induction motor, i.e. of the type comprising a stator 302 and a rotor 304 to which a motor shaft 306 is associated.

In one embodiment, the compressor member 400 comprises a piston 402 connected eccentrically to the motor's drive shaft 306 so as to travel in a cylinder 404 supported by the aerosol body 200. For example, the aerosol body 200 comprises a shell 203, 203' made of plastic material, and the cylinder 404 is made directly in the shell 203. Due to the connection of the piston 402 to the drive shaft 306 by means of a cam, the rotation of the drive shaft 306 results in a substantially rectilinear alternating motion of the piston 402 with respect to the cylinder 404.

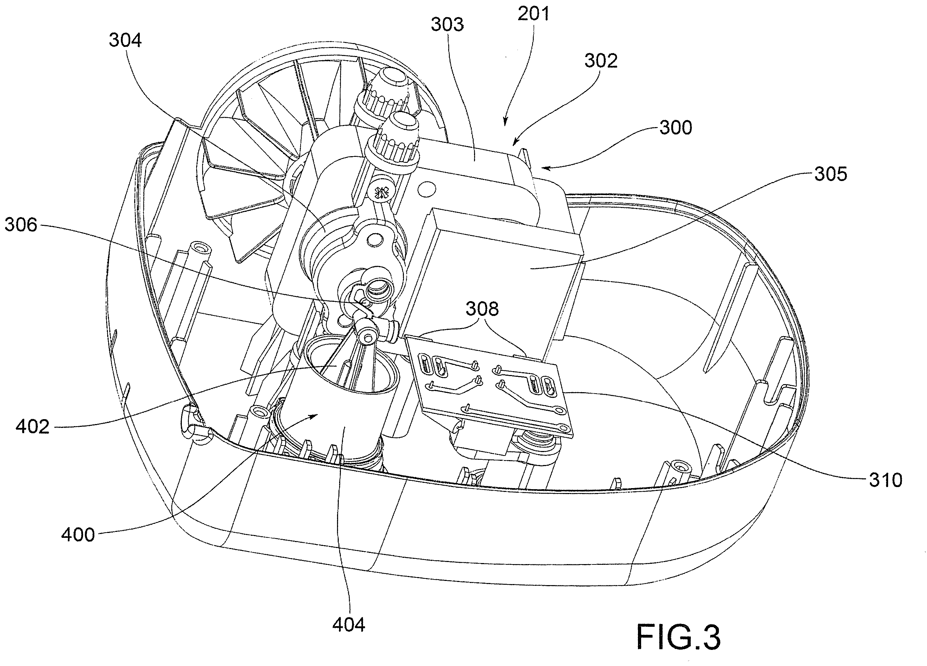

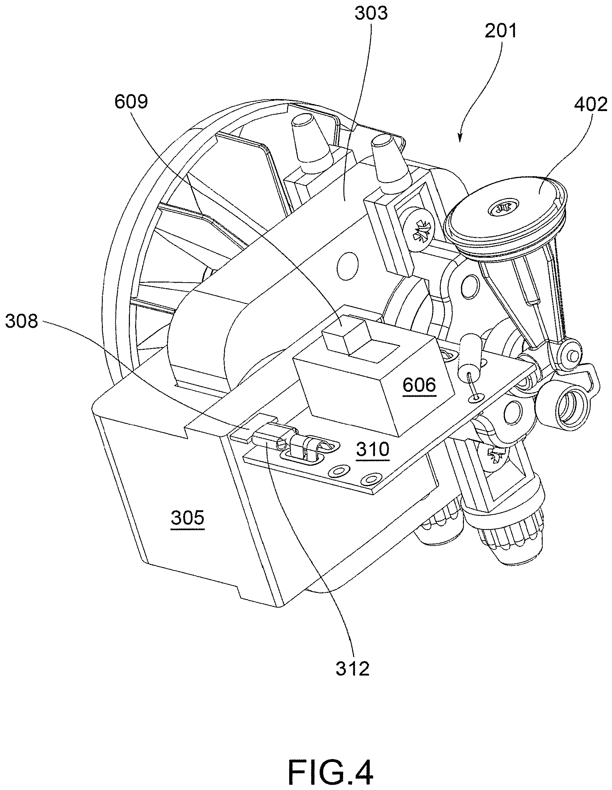

With reference to figures 3 and 4, the stator 302 comprises a laminated core 303 and an electric winding 305. The laminated core 303 has a thickness of less than 18 mm, preferably 15 mm. The electric winding 305 has a number of turns between 1900 and 2500 with the diameter of the copper wire of the turns less than 0.35 mm and preferably 0.31 mm.

The electric motor according to the invention uses between 80 and 100 VA of power, preferably about 90 VA. For example, at 230 V mains voltage, the electric motor uses a current of about 0.4 A or even less, for a power consumption of about 88 VA.

In one embodiment, the compressor member 400 is connected to the electric motor by means of a drive shaft 306 having a diameter between 2 and 4 mm.

In one embodiment, the suction and air delivery means comprise suction passages 406 and delivery passages 408 adapted to fluidically connect the cylinder chamber 404 respectively with the outside environment and with a delivery connector 410 provided, for example, on the shell 203 and connectable to a delivery tube 202 to which the ampoule 1 is attached. Each of these suction and delivery passages is associated with a non-return valve.

The ampoule 1 comprises a cup-shaped ampoule body 10. The ampoule body 10 thus comprises a bottom wall 12 and a side wall 14 which jointly delimit an ampoule chamber 16 for receiving a medication.

In the ampoule body 10 a nozzle 18 is provided suitable to be fluidically connected to the compressor member, for example via the delivery tube. The nozzle 18 has an upper portion 18' extending from the bottom wall 12 into the chamber 16 and a lower portion 18" extending inferiorly from the bottom wall 12 so that it is connectable to the delivery tube 202.

In one embodiment, the upper portion 18' has a conical shape.

The nozzle 18 terminates at the top with a compressed air delivery hole 20.

The ampoule 1 further comprises a nozzle-cover 22 suitable to be attached to the nozzle 18 so as to form with the nozzle 18 at least one suction channel 24 of the medication towards the dispensing hole 20. The suction of the medication through the suction channel 24 occurs by means of the Venturi effect.

In other words, the nozzle 18 is crossed by a duct 19 with a decreasing cross-section which, when a highspeed air stream passes through it, creates low pressure toward the delivery hole 20. Due to such low pressure, particles or drops of the medication, which are at a lower level than the delivery hole, are sucked up along the suction channel 24 until they reach the delivery hole 20.

The nozzle-cover 22 is provided with a nebulizer element 25 positioned in front of and coaxially to the delivery hole 24 to nebulize by impact the air-medication mixture exiting from the delivery hole 20.

The ampoule 1 is further provided with a delivery lid 50 suitable to close superiorly the ampoule body 10 coupled to it, for example, by screwing, pressure, a bayonet connection, or other releasable connection systems.

The delivery lid 50 is provided with a delivery mouth 52 of the nebulized medication or mouthpiece, and a nebulized substance inlet chimney 54 extending into the ampoule body 10 coaxially to the nozzle 18 between an upper end communicating with the delivery mouth 52 and a lower end facing the nozzle-cover 22. In one embodiment, the chimney 54 is a cylindrical duct.

In accordance with an aspect of the invention, the delivery hole 20 has a diameter greater than 0.55 mm, preferably between 0.57 and 0.7 mm.

In addition, the ampoule chamber 16 has an internal volume greater than 90 cm³, for example 110 cm³.

Indeed, it has been found that combining the electro-mechanical features of the electric motor described above with the geometric features of the ampoule results in a marked reduction in the size of the electric motor, mainly due to the reduced thickness of the laminated core 303, and lower power usage; however, this does not negatively affect the nebulization function of the medication and therefore the efficacy of the treatment.

In fact, the increase in the diameter of the delivery hole 20, with respect to the diameter of the delivery hole currently used, allows the use of a lower power electric motor without causing overheating problems. The greater drop in pressure and flow due to the expansion of the delivery hole is, however, compensated by the greater volume of the ampoule chamber. In fact, compared to a smaller volume ampoule chamber, the larger volume also allows the particles to remain suspended in the air for longer before they fall onto the bottom wall of the ampule, and therefore are inhaled by the patient. Therefore, the amount of nebulized substance inhaled by the patient remains substantially unchanged with respect to a conventional ampoule and the treatment is equally effective.

In accordance with an aspect of the invention, the nozzle-cover 22 is provided with at least two radial stop portions 222, 224 spaced axially between them and having a decreasing radial extension from the bottom to the top. Each of these radial stop portions 222, 224 is suitable to be axially engaged from the lower end of a respective chimney 54.

Therefore, when the ampoule is in operation and the compressed air exiting the delivery hole 20 acts on the nebulizer element 25 and then tends to move the nozzle-cover 22 by pushing it upward, the lower end of the chimney 54, being integral with the ampoule body 10, axially locks the nozzle-cover 22, thereby keeping it in position and ensuring that the correct distance between the delivery hole 20 and the nebulizer element 25 is maintained.

In a preferred embodiment, even when the ampoule is not in operation, the bottom end of the chimney 54 abuts against a respective radial portion 222, 224.

Due to the presence of at least two axially spaced stop portions 222, 224, with the same nozzle-cover 22, many delivery lids 50 having chimneys 54 of different lengths may also be used.

Due to the decreasing radial extension of the radial stop portions 222, 224, the greater length chimney 54 may rest on the lower radial stop portion 222 without interfering with the upper radial stop portions 224.

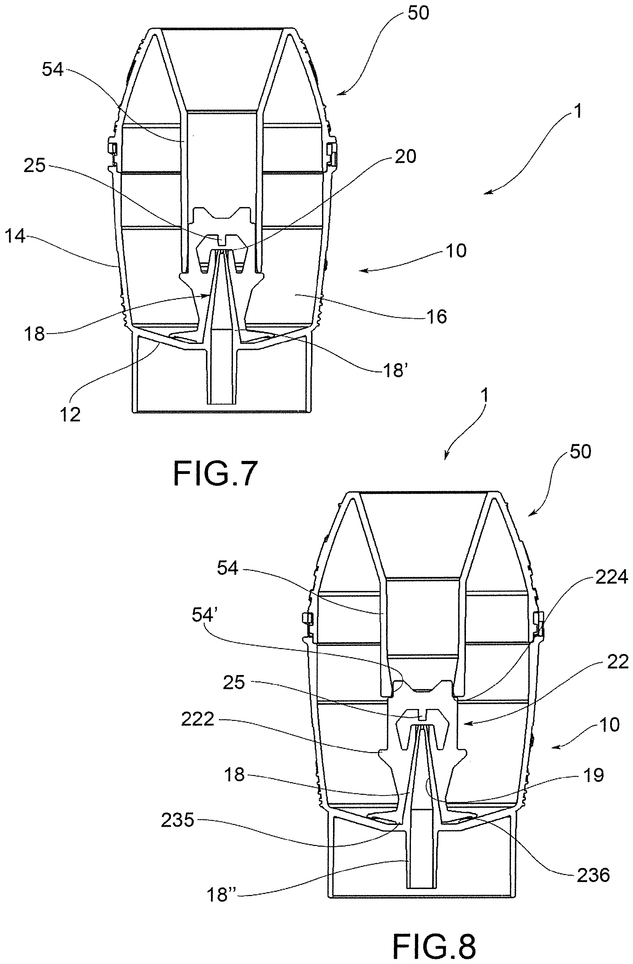

In one embodiment, at least one lower radial portion 222 is made below the level of the delivery hole (figure 7) and at least one upper radial portion 224 is made above the level of the delivery hole (figure 8).

In an embodiment illustrated in figures 7 and 8, the chimneys 54 of different lengths of two different delivery lids have the same inner and outer diameter. However, the shortest chimney 54 has, at the lower end, a radial projection 54' facing inward engaging the relative radial stop portion 224.

In one embodiment, each radial stop portion 222, 224 is formed of a pair of diametrically opposed radial arms.

In one embodiment, the nozzle-cover 22 comprises a conical body 226 from the side wall of which extend radial stop portions 222, 224.

For example, the nozzle-cover 22 comprises a transverse portion 228 extending above the delivery hole 20. The nebulizer element 25 is formed of an axial projection extending inferiorly from said transverse portion 228.

In one embodiment, the nozzle-cover 22 comprises a central body 226 suitable to be fitted on the nozzle 18 and a crown 230 comprising two lateral arms 232 extending upwardly from the side wall of the central body 226 and which are connected, above the delivery hole, by a transverse portion 228. The radial stop portions 222, 224 extend from these side arms 232, while the nebulizer element 25 is supported by the transverse portion 228.

In accordance with another aspect of the invention, the nozzle-cover 22 is provided with a plate-shaped base 234 which is superimposed, at least partially, on the bottom wall 12 of the ampoule body 10. For example, the plate-shaped base 234 covers an annular portion of the bottom wall 12 extending around the delivery nozzle 18.

Preferably, in the case of axially symmetrically shaped ampoules, the plate-shaped base 234 extends at least up to half the radius of the bottom wall 12.

In the case of a concave-shaped bottom wall, the plate-shaped base 234 may also assume a shape complementary to such bottom wall.

The plate-shaped base 234 is provided with support spacers 235 suitable to form a gap 236 between the lower surface of the plate-shaped base 234 and the underlying bottom wall 12. The height of this gap 236, i.e., the distance between the bottom wall and the lower side of the plate-shaped base, is chosen in such a way as to generate a capillary effect between the molecules of the medication and the facing walls that delimit the gap. Due to this capillary effect, even when the ampoule is inclined and the medication only partially occupies such gap, the entire medication may reach the suction channel 24 and be sucked in towards the delivery hole.

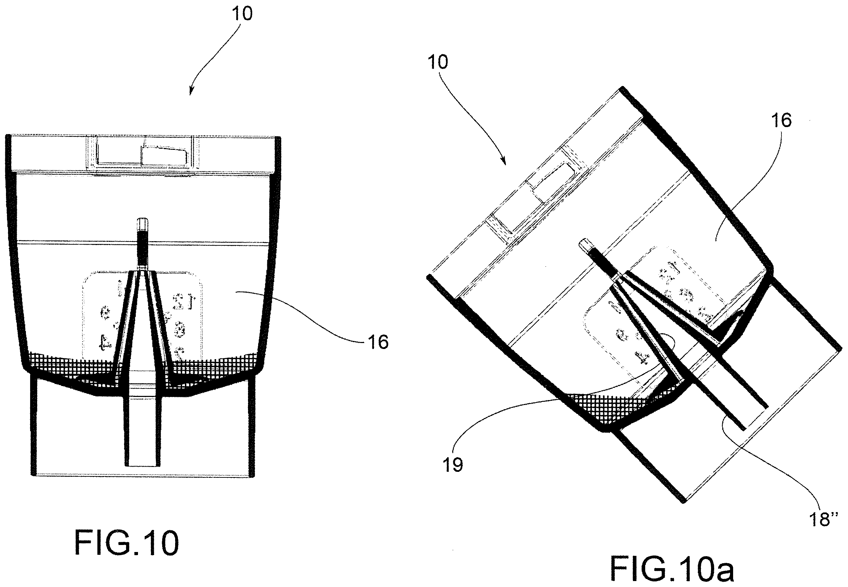

For example, it has been found that with the ampoule according to the invention it is possible to draw out the medication with a 50° inclination of the ampule relative to the vertical (figure 10a).

In accordance with another aspect of the invention, the inner surface of the ampoule chamber 16 is machined by electro-erosion or sandblasting. Thus, a rough surface is obtained which favors the reduction of the surface tension of the drops of the air mixture and the nebulized medication, and therefore said drops fall to the bottom of the chamber, are recuperated and subsequently nebulized. In other words, the rough surface greatly reduces the adhesion of the particles to the side wall of the chamber and consequently the amount of residual substance that is not administered.

This technical arrangement also contributes to maintaining or even improving the effectiveness of the treatment despite the reduction in the size and power used by the electric motor.

In accordance with another aspect of the invention, the delivery mouth 52 is formed in one piece with the delivery lid 50 and is suitable to be connected to an aerosol therapy mask 60 or an accessory for nasal irrigation 70. The integration of the delivery mouth 52 with the delivery lid allows a reduction of the overall internal surface of the ampoule and thereby a reduction of the adhesion of the nebulized medication to such surface and consequently the dispersion of the substance.

This technical arrangement also contributes to maintaining, or even improving, the effectiveness of the treatment despite the reduction in the size and therefore the power used by the electric motor.

Preferably, the delivery mouth 52 has a substantially rectangular cross section, which is more economical to produce.

Returning now to the aerosol body 200, in one embodiment it comprises a shell having an upper portion 203 wherein a knob seat 500 is formed which supports a rotating knob 600 for turning the electric motor 302 on/off.

The knob 600 has a knob head 602 that may be gripped by the user, and a knob shank 604 extending downwards from the knob head to penetrate inside the aerosol body. Such knob shank 604 has a lower end operatively connected to an electric switch 606 for switching the electric motor on/off.

For example, such lower end engages the end of a switch arm 608 which has the opposite end configured to engage a slider 609 of a slide electric switch 606. The knob 600 is therefore rotatable between two positions of the electric motor, on and off, and the rotation of the knob 600 involves a corresponding rotation of the switch arm 608, for example, in a plane orthogonal to that of the rotation axis of the knob 600.

In one embodiment, the knob head 602 joins the knob seat forming a labyrinthine gap 700 extending radially and parallel to the rotation axis of the knob 600 so as to hinder the passage of water from the upper portion of the shell 203 to the electric switch 606, making, for example, the coupling between the knob and the aerosol body to conform at least to the IP21 sealing level.

For example, the knob head 602 has a bowl shape, from the bottom of which a gripping element 610 extends upward that can be gripped by the user. The knob shank 604 extends downward from the inside of the top of the gripping element 610. The knob head 602 has a peripheral edge 612 turned outward radially so as to overlap the upper surface of the shell 203. Such peripheral edge 612 is provided with one or more outer circular teeth 614 engaging the corresponding outer grooves 614' formed in the upper surface of the shell 203.

One or more intermediate circular teeth 615 may be made in the side of the bottom wall of the knob head so as to engage corresponding intermediate grooves 615' made in the knob seat 500.

An additional circular inner tooth 616 may be made in the knob seat 500 to engage an annular cavity 616' extending inside the gripping element 610, between a side wall of this and the knob shank 604.

In one embodiment, in the upper portion 203 of the shell 203, 203', a filter compartment 800 is made wherein a filter 802 is associated with the air suction means. The filter 802 has an upper air inlet end 802' projecting in height with respect to the surface of the upper portion 203 of the shell 203, 203'. In this way, even in the presence of water on the upper surface of the shell, water cannot enter the filter, making for example the coupling between the filter and the aerosol body to conform with at least the IP21 sealing level.

In one embodiment, the filter 802 is protected superiorly by a filter cover 804 and the air penetrates between the filter cover and the filter through an air inlet passage 806 made in the upper portion 203 of the shell 203, 203', alongside the filter body 802.

In one embodiment, the filter cover 804 is provided, for example, around the filter 802 and/or around an opening 805 for the passage of the delivery tube 202, with downwardly projecting protrusions 810 which engage, by calibrated interference, corresponding recesses made in the upper portion 203 of the shell 203, 203'.

Turning now to the electric motor 302, in one embodiment, the electric winding 305 is provided with a pair of male electrical supply connectors 308. The electric motor 302 comprises a printed power and control circuit board 310 secured within the aerosol body and connectable to an electrical power cable. On this circuit board 310 are directly welded female electrical connectors 312 which engage with the male electrical connectors 308.

Therefore, the power and control circuit board 310 connects quickly and safely directly to the winding 305, without the use of wires, and thus makes the motor-compressor group still more compact and inexpensive to manufacture and assemble.

To the embodiments of the device according to the invention, one skilled in the art may, to satisfy contingent needs, make modifications, adaptations and replacements of some elements with others that are functionally equivalent, without departing from the scope of the following claims. Each of the features described as belonging to a possible embodiment may be implemented independently by the other described embodiments.

## Claims

1. An aerosol therapy device (2), comprising an aerosol body (200) wherein a motor-compressor group (201) is housed and wherein suction and air delivery means are provided respectively to and from said motor-compressor group, and an ampoule (1) for the treatment of the respiratory tracts fluidically connected to said air delivery means, the motor-compressor group (201) comprising an electric motor (300) and a compressor member (400) actuatable by the electric motor to suck in and deliver air respectively through said suction and air delivery means, the ampoule (1) comprising a cup-shaped ampoule body (10) defining a chamber (16) for housing a medication and wherein a nozzle (18) having a compressed air delivery hole (20) from the compressor member is provided, and a nozzle-cover (22) suitable to be fitted on the nozzle (18) so as to form with the nozzle at least one channel (24) for the suction of the medication toward the delivery hole (20) by means of the Venturi effect, the nozzle cover (22) being provided with a nebulizer element (25) positioned in front of and coaxially to the delivery hole to nebulize the air-medication mixture exiting from the delivery hole (20), **characterized in that**:
- the electric motor comprises a stator (302) comprising a laminated core (303) having a thickness of less than 18 mm, preferably 15 mm, and an electric winding (305) having a number of turns between 1900 and 2500 with the diameter of the copper wire of the turns less than 0.35 mm and preferably 0.31 mm,
- the compressed air delivery hole (20) has a diameter greater than 0.55 mm, preferably between 0.57 and 0.7 mm,
- the ampoule chamber (16) has an inner volume greater than 90 cm³.

2. A device according to claim 1, wherein the electric motor (300) has a power usage between 80 and 100 VA.

3. A device according to any one of the preceding claims, wherein the compressor member (400) is connected to the electric motor (300) through a shaft (306) with a diameter between 2 and 4 mm.

4. A device according to any one of the preceding claims, wherein the ampoule (1) comprises a delivery lid (50) adapted to close the ampoule body (10) and provided with a delivery mouth (52) of the nebulized medication and a nebulized substance inlet chimney (54) extending into the ampoule body (10) coaxially to the nozzle (18) between an upper end communicating with the delivery mouth (52) and a lower end facing the nozzle-cover (22), and wherein the nozzle-cover is provided with at least two radial stop portions (222, 224) axially spaced between them and having radial extension decreasing from bottom to top, each of said radial stop portions (222, 224) being suitable to be axially engaged from the lower end of a respective chimney (54) .

5. A device according to claim 4, wherein each radial stop portion (222, 224) is formed of a pair of diametrically opposed radial arms.

6. A device according to claim 4 or 5, wherein the nozzle-cover (22) comprises a conical body (226) from the side wall of which the radial stop portions extend.

7. A device according to any one of claims 4-6, wherein the nozzle-cover (22) comprises a transverse portion (228) extending above the delivery hole (20), and wherein the nebulizer element (25) is formed by an axial projection extending inferiorly from said transverse portion.

8. A device according to any one of the claims 4-7, wherein the nozzle-cover (22) comprises a central body (226) suitable to be fitted to the nozzle (18) and a crown (230) comprising two side arms (232) extending upwardly from the side wall of the central body (226) and which are connected above the delivery hole, by a transverse portion (228), the radial stop portions (222, 224) extending from said side arms (232), the nebulizer element (25) being supported by said transverse portion (228).

9. A device according to any one of the claims 4-8, wherein the nozzle-cover is provided with a plate-shaped base (234) which is superimposed on a bottom wall (12) of the ampoule body (10) at least partially, said plate-shaped base being provided with support spacers (235) suitable to form a gap (236) between the bottom surface of the plate-shaped base (234) and the underlying bottom wall (12).

10. A device according to any one of claims 4-9, wherein the delivery mouth (52) is formed in one piece with the delivery lid (50) and is suitable to be connected to an aerosol therapy mask (60) or an accessory for nasal irrigation (70).

11. A device according to the preceding claim, wherein the delivery mouth (52) has a substantially rectangular cross-section.

12. A device according to any one of the preceding claims, wherein the inner surface of the chamber (16) is machined by electro-erosion or sandblasting.

13. A device according to any one of the preceding claims, wherein the aerosol body (200) comprises a shell having an upper portion (203) wherein a knob seat (500) is provided which supports a rotatable knob (600) to turn the electric motor on/off, wherein said knob has a lower end operatively connected to an electric switch (606) and a knob head (602) which joins the knob seat forming a labyrinthine gap (700) extending radially and parallel to the rotation axis of the knob (600) so as to hinder the passage of water from the upper portion of the shell (203) to the electric switch (606) .

14. A device according to any one of the preceding claims, wherein the aerosol body (200) comprises a shell (203, 203') having an upper portion (203) wherein a filter compartment (800) is provided which houses a filter (802) associated with the air suction means, wherein said filter (802) has an upper air inlet end (802') protruding upward with respect to the surface of the upper portion (203) of the shell (203, 203').

15. A device according to the preceding claim, wherein the filter (802) is protected superiorly by a filter cover (804), and wherein the air penetrates between the filter cover and the filter through an air inlet passage (806) made in the upper portion of the shell, alongside the filter body.

16. A device according to any one of the preceding claims, wherein the stator comprises an electric winding (305) provided with a pair of male electrical power connectors (308), wherein the electric motor comprises a printed power and control circuit board (310) connected to an electrical power cable, and wherein on said circuit board are directly soldered female electric connectors (312) which engage with the male electrical connectors (308).

17. A device according to any one of the claims 4-16, comprising at least two interchangeable delivery lids and having chimneys of different heights.

18. A device according to any one of the claims 4-17, comprising an accessory for nasal irrigation which may be coupled to the ampoule delivery mouth.

## Patentansprüche

1. Eine Aerosoltherapievorrichtung (2), umfassend: einen Aerosolkörper (200), in dem eine Motor-Kompressor-Gruppe (201) untergebracht ist und in dem Ansaug- und Luftzufuhrmittel zu bzw. von der genannten Motor-Kompressor-Gruppe vorgesehen sind, und eine Ampulle (1) zur Behandlung der Atemwege, die fluidisch mit den genannten Luftzufuhrmitteln verbunden ist, wobei die Motor-Kompressor-Gruppe (201) einen Elektromotor (300) und ein Kompressorelement (400) umfasst, das durch den Elektromotor betätigt werden kann, um Luft durch die genannten Ansaug- und Luftzufuhrmittel anzusaugen bzw. zuzuführen, wobei die Ampulle (1) einen becherförmigen Ampullenkörper (10) umfasst, der eine Kammer (16) zur Aufnahme eines Medikaments definiert, und in der eine Düse (18) mit einer Abgabeöffnung (20) für Druckluft vom Kompressorelement vorgesehen ist, und eine Düsenabdeckung (22), die dazu geeignet ist, auf die Düse (18) aufgesetzt zu werden, um mit der Düse mindestens einen Kanal (24) für das Ansaugen des Medikaments in Richtung der Abgabeöffnung (20) mittels des Venturi-Effekts zu bilden, wobei die Düsenabdeckung (22) mit einem Zerstäuber-Element (25) versehen ist, das vor und koaxial zur Abgabeöffnung angeordnet ist, um das aus der Abgabeöffnung (20) austretende Luft-Medikamenten-Gemisch zu zerstäuben,
**dadurch gekennzeichnet, dass:**
- der Elektromotor einen Stator (302) umfasst, der einen geschichteten Kern (303) mit einer Dicke von weniger als 18 mm, vorzugsweise 15 mm, und eine elektrische Wicklung (305) mit einer Anzahl von Windungen zwischen 1900 und 2500 umfasst, wobei der Durchmesser des Kupferdrahtes der Windungen weniger als 0,35 mm und vorzugsweise 0,31 mm beträgt,
- die Druckluft-Abgabeöffnung (20) einen Durchmesser von mehr als 0,55 mm, vorzugsweise zwischen 0,57 und 0,7 mm, aufweist,
- die Ampullenkammer (16) ein Innenvolumen von mehr als 90 cm³ hat.

2. Eine Vorrichtung nach Anspruch 1, wobei der Elektromotor (300) eine Leistungsaufnahme zwischen 80 und 100 VA hat.

3. Eine Vorrichtung nach irgendeinem der vorstehenden Ansprüche, wobei das Kompressorelement (400) mit dem Elektromotor (300) über eine Welle (306) mit einem Durchmesser zwischen 2 und 4 mm verbunden ist.

4. Eine Vorrichtung nach irgendeinem der vorstehenden Ansprüche, wobei die Ampulle (1) einen Abgabedeckel (*delivery lid*) (50) umfasst, der zum Verschließen des Ampullenkörpers (10) geeignet ist und mit einer Abgabeöffnung (52) für das vernebelte Medikament und einem Einlasskamin (54) für die vernebelte Substanz versehen ist, der sich in den Ampullenkörper (10) erstreckt, koaxial zur Düse (18) zwischen einem oberen Ende, das mit der Abgabeöffnung (52) in Verbindung steht, und einem unteren Ende, das der Düsenabdeckung (22) zugewandt ist, und wobei die Düsenabdeckung mit mindestens zwei radialen Anschlagabschnitten (222, 224) versehen ist, die axial voneinander beabstandet sind und eine von unten nach oben abnehmende radiale Erstreckung aufweisen, wobei jeder der genannten radialen Anschlagabschnitte (222, 224) geeignet ist, vom unteren Ende eines entsprechenden Kamins (54) aus axial in Eingriff gebracht zu werden.

5. Eine Vorrichtung nach Anspruch 4, wobei jeder radiale Anschlagabschnitt (222, 224) aus einem Paar diametral gegenüberliegender radialer Arme gebildet ist.

6. Eine Vorrichtung nach Anspruch 4 oder 5, wobei die Düsenabdeckung (22) einen konischen Körper (226) umfasst, von dessen Seitenwand die radialen Anschlagabschnitte ausgehen.

7. Eine Vorrichtung nach irgendeinem der Ansprüche von 4 bis 6, wobei die Düsenabdeckung (22) einen Querabschnitt (228) umfasst, der sich oberhalb der Abgabeöffnung (20) erstreckt, und wobei das Zerstäuber-Element (25) durch einen axialen Vorsprung gebildet ist, der sich vom genannten Querabschnitt aus nach unten erstreckt.

8. Eine Vorrichtung nach irgendeinem der Ansprüche von 4 bis 7, wobei die Düsenabdeckung (22) Folgendes umfasst: einen zentralen Körper (226), der geeignet ist, auf die Düse (18) aufgesetzt zu werden, und eine Krone (230), die zwei Seitenarme (232) umfasst, die sich von der Seitenwand des zentralen Körpers (226) nach oben erstrecken und die oberhalb der Abgabeöffnung durch einen Querabschnitt (228) verbunden sind, wobei sich die radialen Anschlagabschnitte (222, 224) von den genannten Seitenarmen (232) erstrecken, wobei das Zerstäuber-Element (25) vom genannten Querabschnitt (228) getragen wird.

9. Eine Vorrichtung nach irgendeinem der Ansprüche von 4 bis 8, wobei die Düsenabdeckung mit einer plattenförmigen Basis (234) versehen ist, die zumindest teilweise auf eine Bodenwand (12) des Ampullenkörpers (10) aufgesetzt ist, wobei die genannte plattenförmige Basis mit Stützabstandshaltern (235) versehen ist, die dazu geeignet sind, einen Spalt (236) zwischen der Bodenfläche der plattenförmigen Basis (234) und der darunter liegenden Bodenwand (12) zu bilden.

10. Eine Vorrichtung nach irgendeinem der Ansprüche von 4 bis 9, wobei die Abgabeöffnung (52) einstückig mit dem Abgabedeckel (50) ausgebildet ist und dazu geeignet ist, mit einer Aerosoltherapiemaske (60) oder einem Zubehörteil zur Nasenspülung (70) verbunden zu werden.

11. Eine Vorrichtung nach dem vorhergehenden Anspruch, wobei die Abgabeöffnung (52) einen im Wesentlichen rechteckigen Querschnitt aufweist.

12. Eine Vorrichtung nach irgendeinem der vorstehenden Ansprüche, wobei die Innenfläche der Kammer (16) durch Elektroerosion oder Sandstrahlen bearbeitet ist.

13. Eine Vorrichtung nach irgendeinem der vorstehenden Ansprüche, wobei der Aerosolkörper (200) ein Gehäuse mit einem oberen Abschnitt (203) umfasst, in dem ein Knopf-Sitz (500) bereitgestellt wird, der einen drehbaren Knopf (600) zum Ein- und Ausschalten des Elektromotors trägt, wobei der genannte Knopf ein unteres Ende aufweist, das mit einem elektrischen Schalter (606) wirkverbunden ist, und einen Knopf-Kopf (602), der mit dem Knopf-Sitz verbunden ist und einen labyrinthartigen Spalt (700) bildet, der sich radial und parallel zur Drehachse des Knopfes (600) erstreckt, um den Durchgang von Wasser vom oberen Teil des Gehäuses (203) zum elektrischen Schalter (606) zu verhindern.

14. Eine Vorrichtung nach irgendeinem der vorstehenden Ansprüche, wobei der Aerosolkörper (200) ein Gehäuse (203, 203') umfasst, das einen oberen Abschnitt (203) aufweist, in dem ein Filterfach (800) vorgesehen ist, das einen dem Luftansaugmittel zugeordneten Filter (802) aufnimmt, wobei der genannte Filter (802) ein oberes Lufteinlass-Ende (802') aufweist, das relativ zur Oberfläche des oberen Abschnitts (203) des Gehäuses (203, 203') nach oben vorsteht.

15. Eine Vorrichtung nach dem vorhergehenden Anspruch, wobei der Filter (802) oben durch eine Filterabdeckung (804) geschützt ist, und wobei die Luft zwischen der Filterabdeckung und dem Filter durch einen Lufteinlasskanal (806) eindringt, der im oberen Teil des Gehäuses entlang des Filterkörpers hergestellt ist.

16. Eine Vorrichtung nach irgendeinem der vorstehenden Ansprüche, wobei der Stator eine elektrische Wicklung (305) umfasst, die mit einem Paar elektrischen Steckverbindern (308) versehen ist, wobei der Elektromotor eine gedruckte Strom- und Steuerplatine (310) umfasst, die mit einem elektrischen Stromkabel verbunden ist, und wobei auf der genannten Platine direkt gelötete elektrische Buchsen (312) vorhanden sind, die mit den elektrischen Steckverbindern (308) in Eingriff stehen.

17. Eine Vorrichtung nach irgendeinem der Ansprüche von 4 bis 16, die mindestens zwei austauschbare Abgabedeckel umfasst und Kamine mit unterschiedlichen Höhen aufweist.

18. Eine Vorrichtung nach irgendeinem der Ansprüche von 4 bis 17, die ein Zubehörteil zur Nasenspülung umfasst, das mit der Ampullen-Abgabeöffnung verbunden werden kann.

## Revendications

1. Dispositif de thérapie par aérosol (2), comprenant un corps d'aérosol (200) dans lequel un groupe moteur-compresseur (201) est logé et dans lequel des moyens d'aspiration et de distribution d'air sont prévus respectivement vers ledit groupe moteur-compresseur et depuis celui-ci, et une ampoule (1) destinée au traitement des voies respiratoires raccordée de manière fluidique auxdits moyens de distribution d'air, le groupe moteur-compresseur (201) comprenant un moteur électrique (300) et un organe compresseur (400) actionnable par le moteur électrique pour aspirer et distribuer de l'air respectivement à travers lesdits moyens d'aspiration et de distribution d'air, l'ampoule (1) comprenant un corps d'ampoule en forme de coupelle (10) définissant une chambre (16) destinée à loger un médicament et dans lequel une buse (18) ayant un orifice de distribution d'air comprimé (20) à partir de l'organe compresseur est prévue, et un capot de buse (22) adapté pour être monté sur la buse (18) de manière à former avec la buse au moins un canal (24) pour l'aspiration du médicament vers l'orifice de distribution (20) au moyen de l'effet Venturi, le capot de buse (22) étant pourvu d'un élément nébuliseur (25) positionné devant l'orifice de distribution et de manière coaxiale à celui-ci pour nébuliser le mélange air-médicament sortant de l'orifice de distribution (20), **caractérisé en ce que** :
- le moteur électrique comprend un stator (302) comprenant un noyau laminé (303) ayant une épaisseur inférieure à 18 mm, de préférence 15 mm, et un enroulement électrique (305) ayant un nombre de tours compris entre 1900 et 2500 avec le diamètre du fil de cuivre des tours étant inférieur à 0,35 mm et de préférence 0,31 mm,
- l'orifice de distribution d'air comprimé (20) a un diamètre supérieur à 0,55 mm, de préférence compris entre 0,57 et 0,7 mm,
- la chambre d'ampoule (16) a un volume intérieur supérieur à 90 cm³.

2. Dispositif selon la revendication 1, dans lequel le moteur électrique (300) a une utilisation de puissance comprise entre 80 et 100 VA.

3. Dispositif selon l'une quelconque des revendications précédentes, dans lequel l'organe compresseur (400) est raccordé au moteur électrique (300) à travers un arbre (306) d'un diamètre compris entre 2 et 4 mm.

4. Dispositif selon l'une quelconque des revendications précédentes, dans lequel l'ampoule (1) comprend un bouchon de distribution (50) adapté pour fermer le corps d'ampoule (10) et pourvu d'une bouche de distribution (52) du médicament nébulisé et d'une cheminée d'entrée de substance nébulisée (54) s'étendant dans le corps d'ampoule (10) de manière coaxiale à la buse (18) entre une extrémité supérieure communiquant avec la bouche de distribution (52) et une extrémité inférieure faisant face au capot de buse (22), et dans lequel le capot de buse est pourvu d'au moins deux parties de butée radiales (222, 224) espacées de manière axiale entre elles et ayant une extension radiale décroissante à partir de la partie inférieure jusqu'à la partie supérieure, chacune desdites parties de butée radiales (222, 224) étant adaptée pour venir en prise de manière axiale à partir de l'extrémité inférieure d'une cheminée respective (54).

5. Dispositif selon la revendication 4, dans lequel chaque partie de butée radiale (222, 224) est formée d'une paire de bras radiaux diamétralement opposés.

6. Dispositif selon la revendication 4 ou 5, dans lequel le capot de buse (22) comprend un corps conique (226) à partir de la paroi latérale de laquelle s'étendent les parties de butée radiales.

7. Dispositif selon l'une quelconque des revendications 4 à 6, dans lequel le capot de buse (22) comprend une partie transversale (228) s'étendant au-dessus de l'orifice de distribution (20), et dans lequel l'élément nébuliseur (25) est formé par une saillie axiale s'étendant de manière inférieure à partir de ladite partie transversale.

8. Dispositif selon l'une quelconque des revendications 4 à 7, dans lequel le capot de buse (22) comprend un corps central (226) adapté pour être monté sur la buse (18) et une couronne (230) comprenant deux bras latéraux (232) s'étendant vers le haut à partir de la paroi latérale du corps central (226) et qui sont raccordés au-dessus de l'orifice de distribution, par une partie transversale (228), les parties de butée radiales (222, 224) s'étendant à partir desdits bras latéraux (232), l'élément nébuliseur (25) étant supporté par ladite partie transversale (228).

9. Dispositif selon l'une quelconque des revendications 4 à 8, dans lequel le capot de buse est pourvu d'une base en forme de plaque (234) qui est superposée sur une paroi inférieure (12) du corps d'ampoule (10) au moins partiellement, ladite base en forme de plaque étant pourvue d'entretoises de support (235) adaptées pour former un espace (236) entre la surface inférieure de la base en forme de plaque (234) et la paroi inférieure sous-jacente (12).

10. Dispositif selon l'une quelconque des revendications 4 à 9, dans lequel la bouche de distribution (52) est formée d'un seul tenant avec le bouchon de distribution (50) et est adaptée pour être raccordée à un masque de thérapie par aérosol (60) ou à un accessoire pour une irrigation nasale (70).

11. Dispositif selon la revendication précédente, dans lequel la bouche de distribution (52) a une section transversale sensiblement rectangulaire.

12. Dispositif selon l'une quelconque des revendications précédentes, dans lequel la surface intérieure de la chambre (16) est usinée par électroérosion ou sablage.

13. Dispositif selon l'une quelconque des revendications précédentes, dans lequel le corps d'aérosol (200) comprend une coque ayant une partie supérieure (203) dans lequel est prévu un siège de bouton (500) qui supporte un bouton rotatif (600) pour activer/désactiver le moteur électrique, dans lequel ledit bouton a une extrémité inférieure raccordée de manière fonctionnelle à un commutateur électrique (606) et une tête de bouton (602) qui rejoint le siège de bouton en formant un espace labyrinthique (700) s'étendant de manière radiale et parallèle à l'axe de rotation du bouton (600) de manière à empêcher le passage de l'eau à partir de la partie supérieure de la coque (203) jusqu'au commutateur électrique (606).

14. Dispositif selon l'une quelconque des revendications précédentes, dans lequel le corps d'aérosol (200) comprend une coque (203, 203') ayant une partie supérieure (203) dans lequel est prévu un compartiment de filtre (800) qui loge un filtre (802) associé aux moyens d'aspiration d'air, dans lequel ledit filtre (802) a une extrémité d'entrée d'air supérieure (802') faisant saillie vers le haut par rapport à la surface de la partie supérieure (203) de la coque (203, 203').

15. Dispositif selon la revendication précédente, dans lequel le filtre (802) est protégé de manière supérieure par un capot de filtre (804), et dans lequel l'air pénètre entre le capot de filtre et le filtre à travers un passage d'entrée d'air (806) réalisé dans la partie supérieure de la coque, à côté du corps de filtre.

16. Dispositif selon l'une quelconque des revendications précédentes, dans lequel le stator comprend un enroulement électrique (305) pourvu d'une paire de raccords de puissance électrique mâles (308), dans lequel le moteur électrique comprend une carte de circuit imprimé de puissance et de commande (310) raccordé à un câble de puissance électrique, et dans lequel sur ladite carte de circuit sont directement soudés des raccords électriques femelles (312) qui viennent en prise avec les raccords électriques mâles (308).

17. Dispositif selon l'une quelconque des revendications 4 à 16, comprenant au moins deux couvercles de distribution interchangeables et ayant des cheminées de hauteurs différentes.

18. Dispositif selon l'une quelconque des revendications 4 à 17, comprenant un accessoire destiné à l'irrigation nasale qui peut être couplé à la bouche de distribution d'ampoule.
